# FASCICULE DE BREVET EUROPEEN

(11) **EP 1 138 954 B1**
(45) Date de publication et mention de la délivrance du brevet: **16.12.2009**
(21) Numéro de dépôt: 01400803.1
(22) Date de dépôt: 28.03.2001
(51) Int. Cl.: F04D 29/42, F04D 25/06, F04D 29/28

(54) **Ventilateur centrifuge**
Kreisellüfter
Centrifugal fan

(30) Priorité: 30.03.2000 FR 0004068
(43) Date de publication de la demande: 04.10.2001
(60) Demande divisionnaire: 09175343.4
(73) Titulaire: TECHNOFAN, 31700 Blagnac (FR)
(72) Inventeur: Darnis, Olivier, 31150 Brugieres (FR); Fontalbat, Thierry, 31120 Portet sur Garonne (FR)
(74) Mandataire: Jacobson, Claude

(56) Documents cités:
- EP-A- 0 487 141
- WO-A-91/17361
- US-A- 2 796 836
- US-A- 2 830 755
- US-A- 5 363 674
- US-A- 5 639 217
- US-A- 5 924 847

## Description

La présente invention concerne un ventilateur centrifuge à haute vitesse, du type comportant un carter définissant intérieurement une volute de guidage du flux gazeux, une roue à aubes d'entraînement du flux gazeux montée rotative dans la volute, et des moyens d'entraînement de la roue à aubes à une vitesse supérieure à 25.000 tours par minute, la roue à aubes présentant, autour de son axe, une bouche d'aspiration du flux gazeux s'étendant en regard d'une entrée d'aspiration ménagée dans le carter et, à sa périphérie, un passage de refoulement du flux gazeux de manière centrifuge suivant un plan de sortie de la roue, le carter présentant en outre une sortie de refoulement du flux gazeux en dehors de la volute.

Pour les malades souffrant d'insuffisance respiratoire, il est connu des machines d'assistance respiratoire permettant d'insuffler aux malades de l'air ambiant au travers d'un masque maintenu en regard des voies respiratoires du patient.

Une telle machine d'assistance respiratoire comporte un ventilateur de mise en mouvement du flux d'air. Ce flux d'air est acheminé jusqu'au masque appliqué sur le visage du patient par des tuyauteries adaptées.

Les ventilateurs utilisés dans les machines d'assistance respiratoire sont des ventilateurs de petite taille ayant un coût de fabrication réduit. Ils fonctionnent généralement à une vitesse de rotation de l'ordre de 10.000 à 20.000 tours par minute.

On constate que les machines d'assistance respiratoire émettent, en fonctionnement, un bruit important qui occasionne des nuisances sonores pour le patient.

Le bruit émis provient essentiellement du ventilateur utilisé pour mettre l'air en mouvement.

Dans un domaine distinct de celui de l'invention, US-A-5, 924, 847 propose un compresseur frigorifique centrifuge, dont les performances de compression de gaz frigorigènes sont améliorées grâce à des paliers magnétiques.

L'invention a pour but de proposer un ventilateur de taille très réduite pouvant être implanté dans une machine d'assistance respiratoire et dont les émissions sonores sont réduites.

A cet effet, l'invention a pour objet un ventilateur centrifuge à haute vitesse du type précité, caractérisé en ce que la volute définit, à sa périphérie, un canal de guidage du flux gazeux qui est essentiellement symétrique en section par rapport au plan de sortie de ladite roue.

Suivant des modes particuliers de réalisation, le ventilateur comporte l'une ou plusieurs des caractéristiques suivantes :
- la roue comporte, des aubes de part et d'autre desquelles sont ménagées des parois pleines annulaires, les parois pleines annulaires et les aubes définissant des conduits disjoints de guidage du flux gazeux ;
- chaque aube définit une surface gauche dont la normale varie suivant trois directions orthogonales le long de la longueur de l'aube ;
- le taux de compression est compris entre 1,05 et 1,12 ;
- la section du canal périphérique de guidage du flux gazeux défini à la périphérie de la volute est progressivement croissante en direction de la sortie de refoulement du flux gazeux en dehors de la volute ;
- le canal périphérique de guidage du flux gazeux défini à la périphérie de la volute décrit extérieurement un contour essentiellement circulaire dans le plan de sortie de la roue, lequel contour circulaire est centré sur l'axe de rotation de la roue ;
- le canal périphérique de guidage du flux gazeux défini à la périphérie de la volute comporte, à sa base, une région sensiblement circulaire en section, et symétrique par rapport au plan de sortie de la roue ;
- la région sensiblement circulaire en section s'étend sur une plage angulaire supérieure à 180° ;
- le bord périphérique de la roue s'étend en dehors de la région sensiblement circulaire en section définie à la base du canal périphérique de guidage du flux gazeux ; et
- l'espace entre le canal périphérique de guidage du flux gazeux et le passage périphérique de refoulement du flux gazeux est dépourvu de tout obstacle pour l'écoulement libre du flux gazeux.

L'invention a également pour objet un dispositif d'assistance respiratoire comportant un bâti dans lequel est monté un ventilateur centrifuge tel que défini ci-dessus et un flexible dont une extrémité est reliée en sortie dudit ventilateur et dont l'autre extrémité est adaptée pour recevoir un masque de ventilation.

L'invention sera mieux comprise à la lecture de la description qui va suivre, donnée uniquement à titre d'exemple et faite en se référant aux dessins, sur lesquels :
- la figure 1 est une vue en coupe longitudinale du ventilateur centrifuge selon l'invention ;

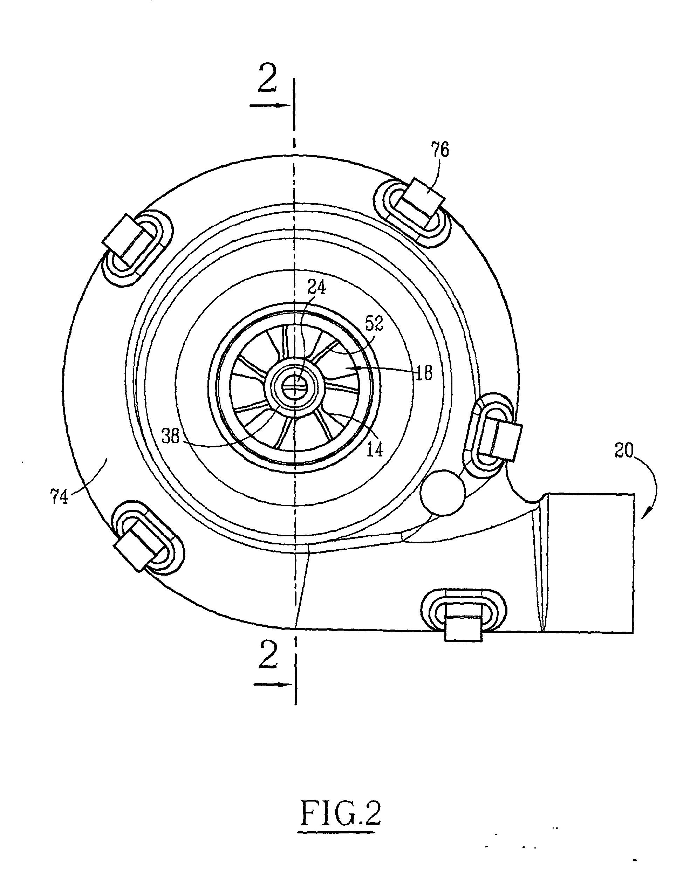

- la figure 2 est une vue en bout du ventilateur selon l'invention ;
- la figure 3 est une vue en perspective de la roue à aubes du ventilateur des figures 1 et 2 ;
- la figure 4 est une vue en élévation de la roue à aubes de la figure 3 dont le flasque a été retiré ; et
- la figure 5 est une vue en coupe du carter du ventilateur de la figure 1 suivant son plan médian dans lequel tourne la roue à aubes.

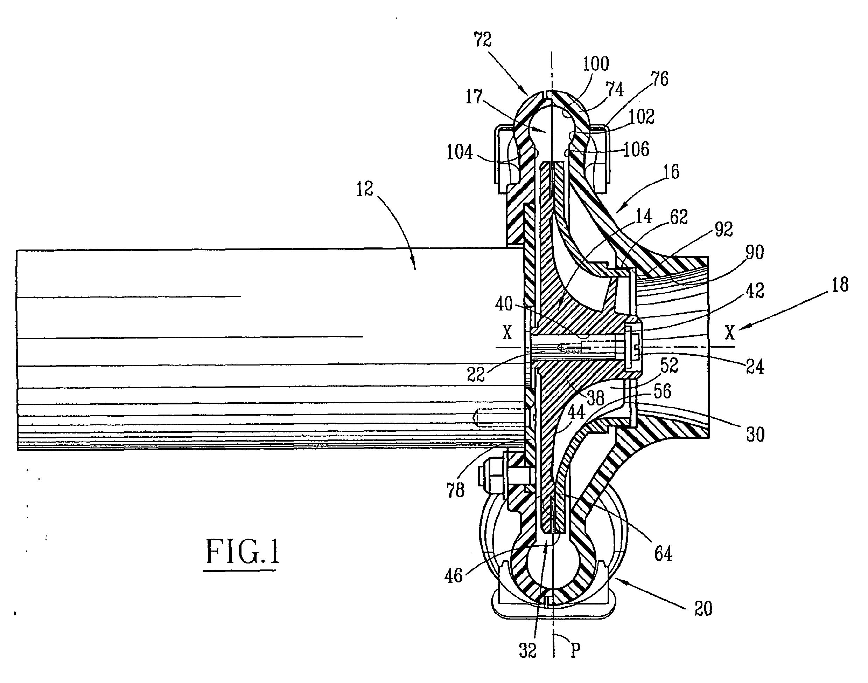

Le ventilateur représenté sur la figure 1 comporte essentiellement un moteur électrique 12, une roue à aubes 14 entraînée par le moteur électrique et un carter 16 délimitant une volute 17 dans laquelle la roue à aubes 14 est montée rotative.

Le carter 16 présente en son centre, suivant l'axe de la roue à aubes 14, une entrée d'aspiration d'air 18. Il présente également à sa périphérie une sortie de refoulement d'air 20 s'étendant tangentiellement à la roue 14.

Le moteur 12 est un moteur électrique de tout type adapté comportant un arbre de sortie 22 d'axe X-X. Le moteur est choisi pour que son arbre de sortie tourne à une vitesse supérieure à 25.000 tours par minute. Cette vitesse est de préférence comprise entre 47.000 et 56.000 tours par minute.

La roue à aubes 14 est fixée directement sur l'arbre de sortie du moteur à l'aide d'une vis 24. Elle est généralement d'axe X-X et est montée rotative autour de cet axe. Ainsi la roue est entraînée à la même vitesse que le moteur 12.

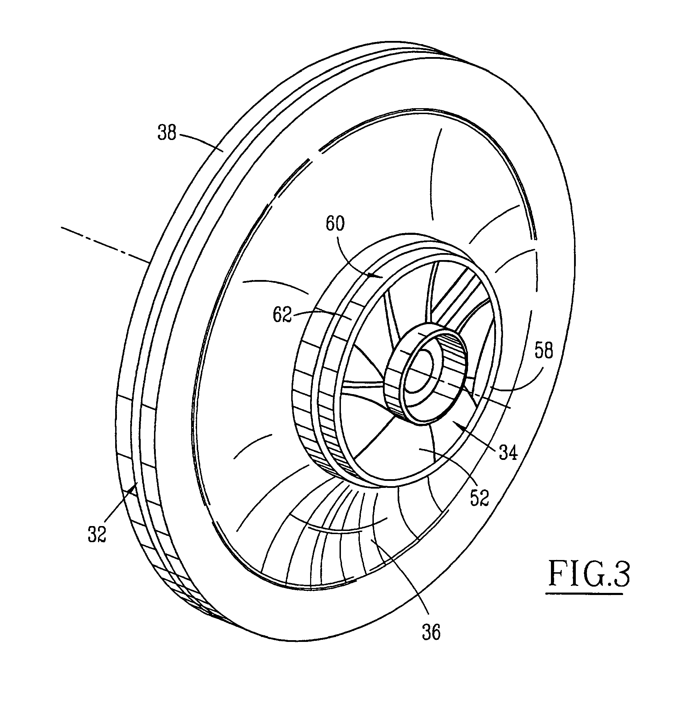

Comme illustré sur la figure 3, la roue à aubes 14 est une roue fermée comportant une bouche d'aspiration axiale 30 ouverte autour de son axe X-X de rotation et un passage périphérique 32 de refoulement centrifuge du flux gazeux ménagé à la périphérie de la roue suivant un plan P dans lequel la roue est entraînée en rotation par le moteur 12. Le passage 32 est ménagé dans l'épaisseur de la roue.

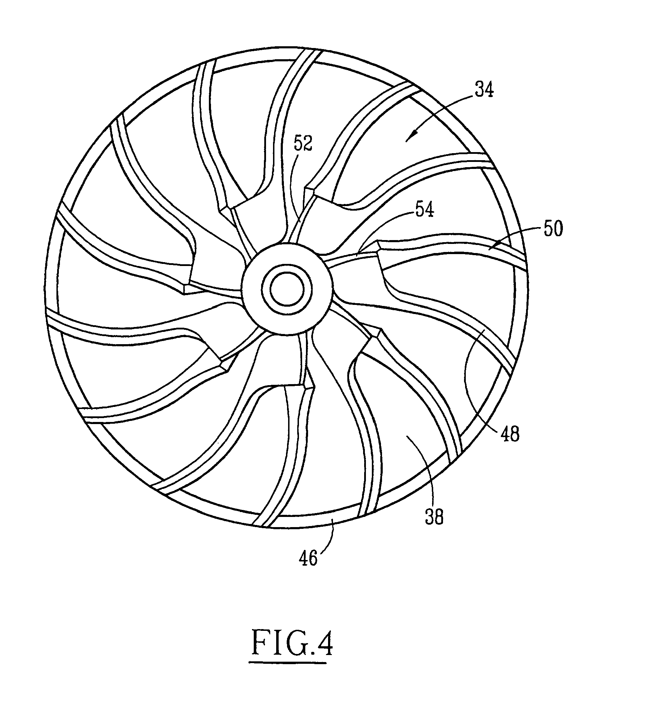

La roue à aubes 14 est formée d'un corps de roue 34 représenté seul sur la figure 4 et d'un flasque annulaire 36 rapporté coaxialement sur le corps de roue.

Le corps de roue 34 et le flasque 36 sont assemblés par exemple par collage ou ultrasons.

Le corps de roue 34 comporte un moyeu 38 traversé axialement par un alésage de réception de l'arbre 22. L'alésage présente à une extrémité un suralésage de réception d'une rondelle d'appui de la tête de vis 24 pour le montage de la roue sur l'arbre 22 du moteur.

Le moyeu 38 est de révolution d'axe X-X. Il présente un diamètre progressivement croissant depuis son extrémité avant délimitant la bouche d'aspiration 30 jusqu'à son extrémité arrière présentant une forme générale de disque et délimitant le passage périphérique de refoulement 32. Ainsi, le moyeu 38 présente une surface latérale 44 de révolution dont le profil s'évase continûment de la bouche d'aspiration 30 au passage périphérique de refoulement 32. Cette surface latérale 44 définit la veine fluide. Elle est constituée par exemple par un tronçon d'hyperboloïde de révolution d'axe X-X.

A son extrémité arrière délimitant le passage périphérique de refoulement 32, la surface 44 présente une plage annulaire plane 46 s'étendant parallèlement au plan P de sortie de la roue.

Comme illustré sur la figure 4, des aubes principales 48 et des aubes secondaires 50 font saillie à la surface latérale 44 de la roue. Ces aubes sont venues de matière avec le moyeu 38 et sont adaptées pour assurer un guidage du flux gazeux de la bouche d'aspiration 30 vers le passage périphérique de refoulement 32.

Les aubes primaires et secondaires sont de hauteur progressivement décroissante depuis la bouche d'aspiration 30 jusqu'au passage périphérique de refoulement 32, la hauteur étant mesurée suivant l'axe X-X.

Les aubes principales 48 présentent un bord d'attaque 52 s'étendant radialement dans la bouche d'aspiration 30 de la roue. Au contraire, les aubes secondaires 50 ont un bord d'attaque 54 disposé en retrait de la bouche d'aspiration 30.

Au-delà de leur bord d'attaque respectif, les profils des aubes 48 et 50 sont identiques jusqu'au passage périphérique de refoulement 32. Ces aubes définissent des surfaces gauches tri-dimensionnelles, c'est-à-dire que la normale à la surface de chaque aube varie continûment dans trois directions orthogonales suivant la longueur de chaque aube.

Le flasque 36 présente une forme extérieure et intérieure généralement tronconique: Sa surface intérieure, notée 56, est adaptée pour s'appliquer exactement sur la surface longitudinale libre des aubes primaires et secondaires. Ainsi, les aubes délimitent entre elles, entre la surface latérale 44 du moyeu et la surface intérieure 56 du flasque rapporté, des canaux indépendants de guidage du flux gazeux. Ces canaux ont une section progressivement croissante de la bouche d'aspiration 30 jusqu'au passage périphérique de refoulement 32.

Le flasque 36 présente, en son centre, un tronçon cylindrique 58 définissant, avec l'extrémité avant du moyeu, la bouche d'aspiration 30. Les bords d'attaque 52 des aubes principales sont entourés par ce tronçon cylindrique 58.

A son extrémité libre, la surface extérieure du tronçon cylindrique 58 présente une surface cylindrique 62 adaptée pour être disposée avec un jeu faible en regard d'un lamage correspondant ménagé dans le carter.

A sa périphérie extérieure, dans la région délimitant le passage périphérique de refoulement 32 de la roue, le flasque 36 présente une plage annulaire plane 64 s'étendant sensiblement parallèlement au plan de sortie P de la roue.

Ainsi, le passage périphérique de refoulement 32 est délimité par les deux plages annulaires 46 et 64 s'étendant presque parallèlement l'une à l'autre. La plage 46 est perpendiculaire à l'axe X-X, alors que la plage 64 est légèrement conique. Ces deux plages assurent le guidage du flux gazeux pour sa sortie de la roue suivant le plan P perpendiculaire à l'axe X-X de rotation.

Le carter 16 comporte deux demi-coquilles 72, 74 reliées l'une à l'autre suivant un plan médian correspondant au plan P de sortie de la roue 14. Les deux demi-coquilles 72 et 74 sont réalisées en matière plastique injectée. Elles sont reliées l'une à l'autre par des agrafes métalliques 76 réparties suivant la périphérie du carter.

La demi-coquille 72 est reliée à la partie fixe du moteur électrique 12 par une plaque 78 qui est vissée à la partie fixe du moteur et sur laquelle est boulonnée la demi-coquille 72.

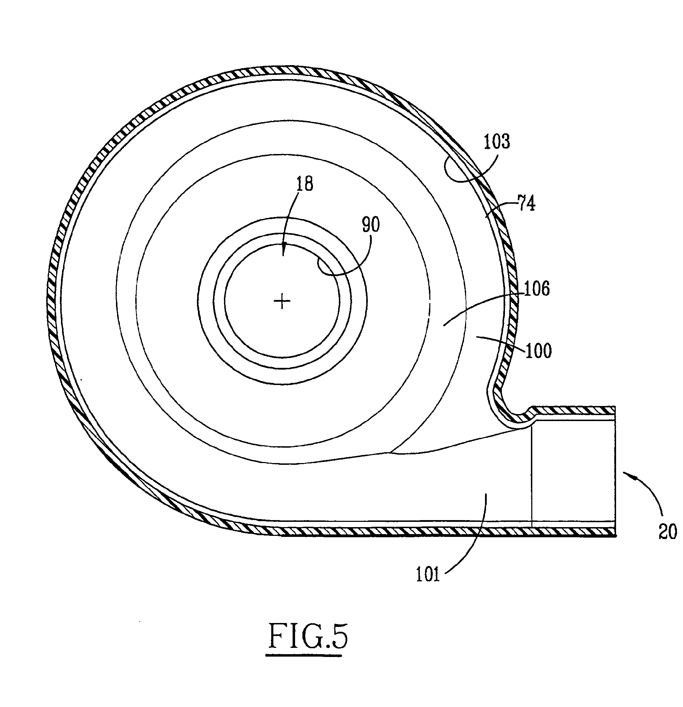

L'entrée d'aspiration 18 est définie dans la demi-coquille 74 représentée seule sur la figure 5. Cette entrée 18 est délimitée par un tronçon convergent 90 s'étendant de l'extrémité ouverte de la demi-coquille 74 jusqu'à la bouche d'aspiration 30 de la roue. En arrière du tronçon d'aspiration 90, la surface intérieure de la demi-coquille 74 comporte un lamage axial 92 qui est appliqué avec un jeu faible en regard de la surface cylindrique 62 de la roue, assurant ainsi l'étanchéité entre le tronçon convergent d'entrée 90 et la volute 17 définie par le carter.

La volute 17 est définie, entre les demi-coquilles 72 et 74. Elle comporte, à la périphérie de la roue 14, en regard du passage périphérique de refoulement 32, un canal circulaire 100 de guidage du flux gazeux.

Comme illustré sur la figure 5, le canal périphérique 100 de guidage du flux gazeux est intérieurement évolutif dans le plan de rotation de la roue. Il définit une spirale centrée sur l'axe X-X de la roue. Le canal 100 s'ouvre sur l'espace médian défini entre les deux demi-coquilles et dans lequel s'étend la roue à aubes 14.

Le canal 100 est relié à la sortie de refoulement par un conduit rectiligne 101 divergeant délimité par les deux demi-coquilles 72, 74. Ce conduit 101 s'étend suivant une direction tangentielle au canal 100. Son diamètre est progressivement croissant du canal 100 vers la sortie de refoulement 20.

Selon l'invention, et comme illustré sur la figure 1, le canal 100 est symétrique en section par rapport au plan P de sortie de la roue. Ce canal présente à sa base une région 102 sensiblement circulaire en section. Cette région sensiblement circulaire est centrée suivant un point du plan P de sortie de la roue. La section de la région sensiblement circulaire 102 augmente progressivement en direction de la sortie de refoulement 20. Elle est croissante sur l'essentiel de la périphérie du carter. Cette région 102 sensiblement circulaire en section s'enroule suivant un contour extérieur 103 circulaire centré sur l'axe X-X. Ainsi, la volute 17 a extérieurement un contour circulaire et intérieurement un contour en spirale. La région 102 s'étend en section sur une plage angulaire supérieure à 180°.

De part et d'autre, la région sensiblement circulaire 102 prévue à la base du canal est prolongée par deux surfaces planes parallèles 104, 106 ménagées respectivement sur les faces intérieures des demi-coquilles 72 et 74. Ces surfaces s'étendent parallèlement au plan P de sortie de la roue.

Le bord extérieur de la roue, dans l'épaisseur duquel est ménagé le passage périphérique de refoulement 32 s'étend en dehors de la région sensiblement circulaire 102 du canal périphérique. Ainsi, le bord extérieur s'étend entre les deux surfaces planes annulaires 104 et 106.

Le canal périphérique 100 de guidage du flux gazeux défini à la périphérie de la volute 17 présente une surface essentiellement lisse et l'espace délimité entre cette surface lisse et le passage périphérique de refoulement 32 de la roue est exempt de tout diffuseur, cet espace étant maintenu libre pour l'écoulement du flux gazeux.

Avantageusement, la roue à aubes 14 est dimensionnée, et notamment ses aubes 48 et 50, de sorte que le taux de compression du ventilateur soit compris entre 1,05 et 1,12.

Dans un tel ventilateur centrifuge, l'air est aspiré par l'entrée d'aspiration 18 et est rejeté par la sortie de refoulement 20.

La vitesse élevée de rotation de la roue 14 et la symétrie du canal périphérique 100 par rapport au plan de sortie des gaz issus de la roue permettent un fonctionnement avec des émissions sonores très réduites, tout en permettant d'assurer un débit satisfaisant de l'ordre de 2,5 l/s avec une élévation de la pression à 70 mbars. Pour un tel débit, le niveau de pression sonore à l'aspiration est de 64 dBA.

De plus, les caractéristiques du ventilateur permettent d'obtenir un fonctionnement stable de celui-ci sur toute la plage de débits.

Un tel ventilateur centrifuge convient ainsi parfaitement pour un dispositif d'assistance respiratoire.

Dans un tel dispositif, le ventilateur est monté dans un bâti. La sortie du ventilateur est reliée à une extrémité d'un flexible, l'autre extrémité du flexible étant adaptée pour recevoir un masque de ventilation.

Un dispositif d'assistance respiratoire équipé d'un tel ventilateur ne crée que de faibles nuisances sonores, améliorant ainsi le confort de l'utilisateur.

## Revendications

1. Ventilateur centrifuge à haute vitesse, du type comportant un carter (16) définissant intérieurement une volute (17) de guidage du flux gazeux, une roue à aubes (14) d'entraînement du flux gazeux montée rotative dans la volute (17), et des moyens (12) d'entraînement de la roue à aubes (14) à une vitesse supérieure à 25.000 tours par minute, la roue à aubes présentant, autour de son axe (X-X), une bouche (30) d'aspiration du flux gazeux s'étendant en regard d'une entrée d'aspiration (18) ménagée dans le carter (16) et, à sa périphérie, un passage (32) de refoulement du flux gazeux de manière centrifuge suivant un plan (P) de sortie de la roue, le carter (16) présentant en outre une sortie (20) de refoulement du flux gazeux en dehors de la volute (17), **caractérisé en ce que** la volute (17) définit, à sa périphérie, un canal (100) de guidage du flux gazeux qui est essentiellement symétrique en section par rapport au plan (P) de sortie de ladite roue.

2. Ventilateur selon la revendication 1, **caractérisé en ce que** la roue comporte, des aubes (48, 50) de part et d'autre desquelles sont ménagées des parois pleines (36, 38) annulaires, les parois pleines annulaires et les aubes définissant des conduits disjoints de guidage du flux gazeux.

3. Ventilateur selon la revendication 1 ou 2, **caractérisé en ce que** chaque aube (48, 50) définit une surface gauche dont la normale varie suivant trois directions orthogonales le long de la longueur de l'aube.

4. Ventilateur selon l'une quelconque des revendications précédentes, **caractérisé en ce que** le taux de compression est compris entre 1,05 et 1,12.

5. Ventilateur selon l'une quelconque des revendications précédentes, **caractérisé en ce que** la section du canal périphérique (100) de guidage du flux gazeux défini à la périphérie de la volute (17) est progressivement croissante en direction de la sortie (20) de refoulement du flux gazeux en dehors de la volute (17).

6. Ventilateur selon l'une quelconque des revendications précédentes, **caractérisé en ce que** le canal périphérique (100) de guidage du flux gazeux défini à la périphérie de la volute (17) décrit extérieurement un contour essentiellement circulaire (103) dans le plan de sortie de la roue (14), lequel contour circulaire (103) est centré sur l'axe (X-X) de rotation de la roue.

7. Ventilateur selon l'une quelconque des revendications précédentes, **caractérisé en ce que** le canal périphérique (100) de guidage du flux gazeux défini à la périphérie de la volute (17) comporte, à sa base, une région (102) sensiblement circulaire en section et, symétrique par rapport au plan P de sortie de la roue.

8. Ventilateur selon la revendication 7, **caractérisé en ce que** la région (102) sensiblement circulaire en section s'étend sur une plage angulaire supérieure à 180°.

9. Ventilateur selon la revendication 6 ou 7, **caractérisé en ce que** le bord périphérique de la roue s'étend en dehors de la région (102) sensiblement circulaire en section définie à la base du canal périphérique de guidage du flux gazeux.

10. Ventilateur selon l'une quelconque des revendications précédentes, **caractérisé en ce que** l'espace entre le canal périphérique (100) de guidage du flux gazeux et le passage périphérique .de refoulement du flux gazeux (32) est dépourvu de tout obstacle pour l'écoulement libre du flux gazeux.

## Claims

1. A high speed centrifugal fan of the kind comprising a casing (16) defining in its interior a spiral (17) for guiding gas flow, a vaned wheel (14) for delivering the gas flow rotatably mounted in the spiral (17), and means (12) for driving the vaned wheel (14) at a speed greater than 25,000rpm, the vaned wheel having, about its axis (X-X), an inlet (30) for drawing in the gas flow extending opposite an aspiration inlet (18) arranged in the housing (16) and, at its periphery, a passage (32) for delivering the gas flow in a centrifugal manner along an outlet plane (P) of the wheel, the casing (16) having in addition an outlet (20) for delivering the gas flow from the spiral (17), **characterised in that** the spiral (17) defines, at its periphery, a passage (100) for guiding the gas flow which is substantially symmetrical in section in relation to the outlet plane (P) of the wheel.

2. A fan according to claim 1, **characterised in that** the wheel comprises vanes (48,50) on both sides of which are arranged solid annular walls (36,38), the solid annular walls and the vanes defining separate passages for guiding the gas flow.

3. A fan according to claim 1 or claim 2, **characterised in that** each vane (48,50) defines a twisted surface whose normal varies according to three orthogonal directions along the length of the vane.

4. A fan according to any one of the preceding claims, **characterised in that** the compression ratio is between 1.05 and 1.12.

5. A fan according to any one of the preceding claims, **characterised in that** the section of the peripheral passage (100) for guiding gas flow defined at the periphery of the spiral (17) increases progressively in the direction of the outlet (20) for delivery of the gas flow outside the spiral (17).

6. A fan according to any one of the preceding claims, **characterised in that** the peripheral passage (100) for guiding gas flow defined at the periphery of the spiral (17) has an essentially circular external outline (103) in the outlet plane of the wheel (14), the circular outline (103) being centred on the axis (X-X) of rotation of the wheel.

7. A fan according to any one of the preceding claims, **characterised in that** the peripheral passage (100) for guiding the gas flow defined at the periphery of the spiral comprises, at its base, a region (102) which is substantially circular in section and symmetrical in relation to the outlet plane (P) of the wheel.

8. A fan according to claim 7, **characterised in that** the region (102) which is substantially circular in section extends for an angular range greater than 180°.

9. A fan according to claim 6 or 7, **characterised in that** the peripheral edge of the wheel extends outside the region (102) which is substantially circular in section and defined at the base of the peripheral passage for guiding the gas flow.

10. A fan according to any one of the preceding claims, **characterised in that** the space between the peripheral passage (100) for guiding the gas flow and the peripheral passage for delivering the gas flow (32) has no obstacles for the free flow of the gas flow.

## Patentansprüche

1. Hochgeschwindigkeits-Zentrifugalgebläse der Bauart, die ein Gehäuse (16), das im Inneren ein Spiralgehäuse (17) zur Führung des Gasstroms bildet, ein Schaufelrad (14) zum Durchziehen des Gasstroms, das drehbeweglich im Spiralgehäuse (17) angebracht ist, und Einrichtungen (12) zum Antrieb des Schaufelrads (14) auf eine Geschwindigkeit von über 25.000 Umdrehungen pro Minute umfasst, wobei das Schaufelrad (14) um seine Achse (X-X) eine Öffnung (30) zum Ansaugen des Gasstroms, die sich gegenüber einem Ansaugeinlass (18) erstreckt, der im Gehäuse (16) ausgebildet ist, und an seinem Umfang einen zentrifugalen Druckdurchlass (32) für den Gasstrom entlang einer Auslassebene (P) des Rads aufweist, wobei das Gehäuse (16) darüber hinaus einen aus dem Spiralgehäuse (17) führenden Druckauslass (20) für den Gasstrom aufweist, **dadurch gekennzeichnet, dass** das Spiralgehäuse (17) an seinem Umfang einen Kanal (100) zur Führung des Gasstroms bildet, welcher Kanal in Bezug auf die Auslassebene des Rads im Schnitt im Wesentlichen symmetrisch ist.

2. Gebläse nach Anspruch 1, **dadurch gekennzeichnet, dass** das Rad Schaufeln (48, 50) umfasst, an deren beiden Seiten massive Ringwände (36, 38) ausgebildet sind, wobei die massiven Ringwände und die Schaufeln voneinander getrennte Kanäle zur Führung des Gasstroms bilden.

3. Gebläse nach Anspruch 1 oder 2, **dadurch gekennzeichnet, dass** jede Schaufel (48, 50) eine linke Fläche bildet, deren Normale in drei orthogonalen Richtungen entlang der Länge der Schaufel variiert.

4. Gebläse nach einem der vorhergehenden Ansprüche, **dadurch gekennzeichnet, dass** das Verdichtungsverhältnis zwischen 1,05 und 1,12 beträgt.

5. Gebläse nach einem der vorhergehenden Ansprüche, **dadurch gekennzeichnet, dass** der Schnitt des am Umfang des Spiralgehäuses (17) gebildeten umfänglichen Kanals (100) zur Führung des Gasstroms in Richtung auf den Druckauslass (20) für den Gasstrom aus dem Spiralgehäuse (17) progressiv zunimmt.

6. Gebläse nach einem der vorhergehenden Ansprüche, **dadurch gekennzeichnet, dass** der am Umfang des Spiralgehäuses (17) gebildete umfängliche Kanal (100) zur Führung des Gasstroms in der Auslassebene des Rads (14) außen eine im Wesentlichen kreisförmige Außenlinie (103) beschreibt, welche kreisförmige Außenlinie (103) an der Drehachse (X-X) des Rads zentriert ist.

7. Gebläse nach einem der vorhergehenden Ansprüche, **dadurch gekennzeichnet, dass** der am Umfang des Spiralgehäuses (17) gebildete umfängliche Kanal (100) zur Führung des Gasstroms an seiner Basis einen im Schnitt im Wesentlichen kreisförmigen und in Bezug auf die Auslassebene P des Rads symmetrischen Abschnitt (102) umfasst.

8. Gebläse nach Anspruch 7, **dadurch gekennzeichnet, dass** der im Schnitt im Wesentlichen kreisförmige Abschnitt (102) sich über einen Winkelbereich von über 180° erstreckt.

9. Gebläse nach Anspruch 6 oder 7, **dadurch gekennzeichnet**, das sich der Umfangsrand des Rads aus dem im Schnitt im Wesentlichen kreisförmigen Bereich (102) nach außen erstreckt, der an der Basis des umfänglichen Kanals zur Führung des Gasstroms gebildet ist.

10. Gebläse nach einem der vorhergehenden Ansprüche, **dadurch gekennzeichnet, dass** der Raum zwischen dem umfänglichen Kanal (100) zur Führung des Gasstroms und dem umfängliche Druckdurchlass (32) für den Gasstrom zum freien Ausströmen des Gasstroms frei von jedem Hindernis ist.
